# EUROPEAN PATENT SPECIFICATION

(11) **EP 0 882 000 B1**
(45) Date of publication and mention of the grant of the patent: **28.08.2002**
(21) Application number: 97906460.7
(22) Date of filing: 30.01.1997
(51) Int. Cl.: C07C 5/00

(54) **HYDROISOMERIZATION WITH REDUCED HYDROCRACKING**
HYDROISMERISIERUNG MIT VERMINDERTEN HYDROKRACKEN
HYDROISOMERISATION A HYDROCRAQUAGE REDUIT

(30) Priority: 02.02.1996 US 595713
(43) Date of publication of application: 09.12.1998
(73) Proprietor: ExxonMobil Research and Engineering Company, Annandale, New Jersey 08801 (US)
(72) Inventor: WITTENBRINK, Robert, Jay, Baton Rouge, LA 70816 (US); RYAN, Daniel, F., Baton Rouge, LA 70820 (US); BAIRD, William, C., Jr., Baton Rouge, LA 70808 (US)
(74) Representative: Dew, Melvyn John
(86) International application number: US9701585
(87) International publication number: WO97028106

(56) References cited:
- WO-A-97/03750
- FR-A- 1 097 445
- FR-A- 1 457 131
- US-A- 3 177 159
- US-A- 3 269 937
- US-A- 3 320 155

## Description

This invention relates to the hydroisomerization of feeds containing heteroatoms, e.g., sulfur or oxygen. More particularly, this invention relates to the hydroisomerization of feeds over a catalyst containing cobalt and molybdenum components and a hydrocracking suppressant.

Hydroisomerization processes generally require the removal, e.g., by hydrotreating, of sulfur and nitrogen compounds that can rapidly deactivate or poison the relatively expensive isomerization process catalyst. Consequently, feeds to be isomerized are first contacted with a sulfur tolerant catalyst in the presence of hydrogen for minimizing the amount of sulfur in the feed.

Additionally, isomerization processes, particularly those carried out in the presence of hydrogen, are effected with unsulfided catalysts. As a consequence, hydrogenolysis, e.g., hydrocracking, occurs early in the processing and produces significant amounts of gaseous products, e.g., methane or C₁-C₄ hydrocarbons. A process, therefore, that can eliminate or substantially reduce the hydrogenolysis aspect of the process can be more efficient and more economic because of increased yields of desired products and decreased yields of gaseous products.

FR-A-1097445 discloses a process for the catalytic isomerization of hydrocarbon mixtures containing at least 20% by weight of paraffinic hydrocarbons, characterised by the following points, separately or in combinations:
1. The starting material is passed, in the presence of hydrogen or of a gas containing hydrogen, at a temperature between 350° and 450°C and preferably between 400° and 440°C, at a pressure of between 10 and 40 atmospheres and preferably between 20 and 40 atmospheres, in contact with a catalyst consisting of one or more metals or compounds, in particular oxides or sulphides, of metals from the sixth group, left-hand column and/or from the eighth group of the Periodic Table, with the exception of the metals from the platinum group, deposited on an acidic support.
2. The catalyst is made acidic or its acidity is increased by introducing a halogen, preferably fluorine.
3. The catalyst consists of a molybdenum/alumina/fluorine catalyst, containing less than 5% by weight of fluorine, uniformly distributed over the active surface of the support.
4. The catalyst is composed of at least 7 parts by weight of molybdenum, calculated in the form of MoO₃ in the form of oxide or sulphide, deposited on an alumina support obtained by precipitation in a solution of an aluminium salt, whereas, during the preparation of the catalyst, HF or a substance which generates HF under the conditions in which the catalyst is prepared, is added in the form of a solution whose concentration does not exceed 1% by weight, calculated in the form of HF or of a diluted gas , in a proportion of less than 1.0 part by weight of fluorine per 100 parts by weight of alumina.

FR-A-1457131 and counterpart GB-A-1065205 both disclose a process for the production of lubricating oils or lubricating oil components from petroleum waxes, which comprises the following steps:
1. Isomerizing a petroleum wax in the presence of hydrogen in a first zone over a catalyst containing a hydrogenation-dehydrogenation component and a solid acid-acting refractory support.
2. Isomerizing at least the portion boiling in the lubricating oil boiling range of the effluent from the first zone in the presence of hydrogen over a catalyst containing a hydrogenation-dehydrogenation component and a solid acid-acting refractory support in a second zone.
3. Separating the portion boiling in the lubricating oil boiling range from the effluent of the second zone.
4. Dewaxing this portion to recover a substantially wax-free lubricating oil or lubricating oil component.

The catalyst in the first zone and/or in the second zone may comprise at least one metal of Groups II, V, VII and VIII of the periodic Table of Elements and/or at least one oxide and/or sulphide of at least one such metal.

US-A-3177159 discloses a process for hydroisomerizing olefins. The process employs a catalyst which comprises nickel and tungsten deposited on an acid-acting support, the atomic ratio of nickel to tungsten being from about 10 to 1 to about 150 to 1, the amount of nickel being from about 0.5 to 20% by weight, based on the total catalyst, and said nickel and tungsten being in the form of a sulphide.

WO-A-97/03750, which was published after the priority date of the present European patent application, discloses a hydroconversion (hydroisomerization) catalyst comprising nickel, in an amount of less than about 15 wt% catalyst and copper in a copper:nickel ratio of less than about 1:2 supported on an amorphous silica-alumina carrier having less than about 30 wt% alumina, the carrier having a surface area of greater than about 200 m2/gm and a pore volume of less than about 1.0 ml/gm.

The present invention provides a hydroisomerization process comprising: contacting a 350°F + (176.7°C+) hydrocarbon-containing feed in the presence of hydrogen and a catalyst having an acidic functionality and comprising a cobalt component, a molybdenum component, and a hydrocracking suppressant selected from copper and/or sulfur component(s).

Hydrocracking suppression can be effectively measured by suppressing methane, since hydrocracking most easily occurs through terminal cracking. The process is conducted with hydrocarbon containing feeds at usual hydroisomerization conditions. Generally, the process of this invention will lead to methane yields of less than about 5 wt% based on total 700°F+ (371°C+) conversion, preferably less than about 2 wt%, more preferably less than about 1 wt%, and still more preferably less than about 0.5 wt%.

Typical hydroisomerization conditions are well known in the literature and can vary widely. For example, broad and preferred ranges for these conditions are shown in the following table:

| CONDITION | BROAD | PREFERRED |
|---|---|---|
| Temperature, °F (°C) | 300-900 (149-482°C) | 550-750 (288-399°C) |
| Total pressure, psig (bar gauge) | 0-2500 (0-172.4) | 300-1200 (20.7-82.8) |
| Hydrogen Treat Rate, SCF/B(l H₂/l feed) | 500-5000 (88.4-889.5) | 2000-4000 (355.8-711.6) |
| Hydrogen Consumption Rate, SCF/B (l/l) | 50-500 (8.9-88.9) | 100-300 (17.8-53.4) |

The catalysts useful in this invention preferably contain an acid function as well as the hydrocracking suppressant. The hydrocracking suppressant is either copper, e.g., in amounts of from 0.1-10wt%, or a source of sulfur, or both. The source of sulfur can be provided by pre-sulfiding the catalyst by known methods, for example, by treatment with hydrogen sulfide until breakthrough occurs. The use of pre-sulfided non-noble metal catalysts for hydroisomerization is believed to be unprecedented.

When feeds that are essentially free of sulfur are processed in accordance with this invention, sulfur may be stripped from a sulfided catalyst As a consequence, it will be preferred to add small amounts of sulfur with the feed, either continuously or periodically to maintain a desired level of sulfur in the catalyst. Catalysts containing sulfur typically have at least about 0.01 wt% sulfur, preferably about 0.01 to 20% sulfur, more preferably 0.1 to 10 wt%.

The cobalt component is usually present in catalytically effective amounts, that is, in a range of from 0.5 to 20 wt%. The molybdenum component may be present in amounts in a range of from 1 to 20 wt%.

The acid functionality can be furnished by a support with which the catalytic metal or metals can be composited in well known methods. The support can be any refractory oxide or mixture of refractory oxides or zeolites or mixtures thereof. Preferred supports include silica, alumina, silica-alumina, silica-alumina-phosphates, titania, zirconia, vanadia and other Group III, IV, V or VI oxides, as well as Y sieves, such as ultra stable Y sieves. Preferred supports include alumina and silica-alumina, more preferably silica-alumina where the silica concentration of the bulk support is less than about 50 wt%, preferably less than about 35 wt%, more preferably 15-30 wt%. When alumina is used as the support, small amounts of chlorine or fluorine may be incorporated into the support to provide the acid functionality.

A preferred supported catalyst has surface areas in the range of from 180 to 400 m²/gm, preferably from 230 to 350 m²/gm, and a pore volume in a range of from 0.3 to 1.0 ml/gm, preferably from 0.35 to 0.75 ml/gm, a bulk density in a range of from 0.5-1.0 g/ml, and a side crushing strength in a range of from 0.8 to 3.5 kg/mm.

The preparation of preferred amorphous silica-alumina microspheres for use as supports is described in Ryland, Lloyd B., Tamele, M.W., and Wilson, J.N., Cracking Catalysts, Catalysis; Volume VII, Ed. Paul H. Emmett, Reinhold Publishing Corporation, New York, 1960.

During hydroisomerization, the 700°F+ (371 deg C+) conversion to 700°F- (371 deg C-) ranges from about 20-80%, preferably from 30-70%, more preferably from about 40-60%; and essentially all olefins and oxygenated products are hydrogenated.

The feed can be any hydrocarbon containing material having a final boiling point of less than about 1050°F (566°C). A particularly preferred feed is a material derived from a non-shifting Fischer-Tropsch process.

The feed materials that are hydroisomerized are typically waxy feeds, C5+, preferably boiling above about 350°F (177°C), more preferably above about 550°F (288°C), and are preferably obtained from a Fischer-Tropsch process which produces substantially normal paraffins, or may be obtained from slack waxes. Slack waxes are the by-products of dewaxing operations where a diluent such as propane or a ketone (e.g., methylethyl ketone, methylisobutyl ketone) or other diluent is employed to promote wax crystal growth, the wax being removed from the lubricating oil by filtration or other suitable means. The slack waxes are generally paraffinic in nature, boil above about 600°F (315°C), preferably in the range of 600°F to 1050°F (315-566°C) and may contain from about 1 to about 35 wt% oil. Waxes with lower oil contents, e.g., 5-20 wt% are preferred; however, waxy distillates or raffinates containing 5-45% wax may also be used as feeds. Slack waxes are usually freed of polynuclear aromatics and hetero-atom compounds by techniques known in the art; e.g., mild hydrotreating as described in U.S. Patent 4,900,707, which also reduces sulfur and nitrogen levels. Feeds which contain high levels of sulfur, e.g., >30 ppm sulfur, may also be used as the catalyst described here is sulfur tolerant. In addition, feeds such as gas field condensates may be used as feeds or other petroleum derived feeds with high sulfur levels that require hydroisomerization to improve their properties. A distillation showing the fractional make up (±10 wt% for each fraction) for a typical Fischer-Tropsch process feed stock follows:

| Boiling Temperature Ranges | Wt% of Fraction |
|---|---|
| IBP-320°F (160°C) | 13 |
| 320-500°F (160-260°C) | 23 |
| 500-700°F (260-371°C) | 19 |
| 700-1050°F (371-566°C) | 34 |
| 1050°F+ (566°C+) | 11 |
| Total | 100 |

The feed may be treated or untreated as regards the removal of hetero-atoms containing compounds, e.g., sulfur and oxygen containing compounds. (Nitrogen does not seem to be a problem, either its presence or absence.) However, when the feed is treated, essentially all of the sulfur and oxygen should be removed, that is, to sulfur levels of less than about 10 wppm, and oxygen levels of less than about 10 wppm. Such feeds are characterized by the substantial absence of sulfur and oxygen. Hydrotleating is effected by any of the well known hydrotreating, e.g., hydrodesulfurization, processes known in the literature.

The catalyst can be prepared by any well known method, e.g., impregnation with an aqueous salt, incipient wetness technique, followed by drying at about 125-150°C for 1-24 hours, calcination at about 300-500°C for about 1-6 hours, reduction by treatment with a hydrogen or a hydrogen containing gas, and, if desired, sulfiding by treatment with a sulfur containing gas, e.g., H₂S at elevated temperatures. The catalyst will then have about 0.01 to 10 wt% sulfur. The metals can be composited or added to the catalyst either serially, in any order, or by co-impregnation of two or more metals.

The following examples will serve to illustrate, but not limit this invention.

### Example 1

A commercial Co-Mo catalyst on a SiO₂-Al₂O₃ support containing 20-30 wt% bulk silica was reduced at 370°C for 3 hours in hydrogen. The catalyst was used to hydroisomerize n-heptane as a model compound representing the more refractory paraffins present in condensate. The results of the isomerization test are found in the following table.

### Example 2

The Co-Mo catalyst of Example 1 was impregnated with an aqueous solution of copper nitrate to introduce 0.3 wt% Cu. The catalyst was calcined in air at 370°C and reduced in hydrogen at 370°C for 3 hours. The Co-Mo-Cu catalyst was used to hydsoisomerize n-heptane. The results are presented in the table below.

### Example 3

The Co-Mo catalyst of Example 1 was reduced in hydrogen at 370°C for 3 hours and breakthrough sulfided with dilute H₂S in H₂ at 370°C. The catalyst was H₂ stripped at the same temperature for 2 hours to remove any chemisorbed H₂S. The Co-Mo-S catalyst was used to hydroisomerize n-heptane. The results are included in the following table.

The catalyst of Example 1, while active for hydroisomerization, has extremely high hydrocracking activity as evidenced by very high methane and n-butane yields and the destruction of normal and isoheptanes. Liquid yield is decreased to a value < 70 wt%.

The catalysts of this invention, Co-Mo-Cu and Co-Mo-S, the catalysts of Examples 2 and 3, are preferred hydroisomerization catalysts on the basis of higher selectivity to isomerized product and substantially decreased hydrocracking activity. In both cases the yields of liquid product exceed 92 wt%, and the formation of isoheptanes is roughly 40% greater than that of Example 1. While not shown in the table, the combination of sulfur with Cu would offer additional yield and selectivity credits relative to those of Examples 2 and 3.

| ISOMERIZATION OF HEPTANE WITH SULFIDED CO-MO AND CO-MO-CU CATALYSTS n-Heptane, 425°C, 100 psig (6.896 bar gauge), 5 W/H/W, H₂/Oil=6 | | | |
|---|---|---|---|
| EXAMPLE | 1 | 2 | 3 |
| Catalyst | Co-Mo | Co-Mo-Cu | Co-Mo-S |
| C₁ | 6.4 | 1.4 | 0.2 |
| i-C₄ | 0.5 | 0.3 | 0.1 |
| n-C₄ | 4.0 | 0.8 | 1.0 |
| n-C₇ | 56.3 | 77.5 | 77.7 |
| 2,4-DMP | 0.4 | 0.6 | 0.4 |
| 2-Me-Hex | 4.6 | 6.2 | 6.5 |
| 3-Me-Hex | 6.4 | 8.6 | 9.6 |
| i-C₇'s | 11.4 | 15.4 | 16.6 |

## Claims

1. A hydroisomerization process comprising: contacting a 350°F+ (176.7°C+) hydrocarbon-containing feed in the presence of hydrogen and a catalyst having an acidic functionality and comprising a cobalt component, a molybdenum component, and a hydrocracking suppressant selected from copper and/or sulfur component(s).

2. The process of claim 1 wherein the sulfur hydrocracking suppressant is provided in the form of, or comprises, a pre-sulfided catalyst.

3. The process of claim 1 or claim 2 wherein the catalyst contains in a range of from 0.01 to 20 wt% sulfur.

4. The process of any one of claims 1 to 3 wherein sulfur is present on the catalyst in an amount in a range of from 0.1 to 10 wt%.

5. The process of any one of claims 1 to 4 wherein sulfur moiety is added to the feed, continuously or periodically, to maintain a desired amount of sulfur component in the catalyst.

6. The process of any one of claims 1 to 5 wherein the cobalt content of the catalyst is in the range of from 0.5 to 20 wt%.

7. The process of any one of claims 1 to 6 wherein the molybdenum content of the catalyst is in the range of from 1.0 to 20 wt%.

8. The process of any one of claims 1 to 7 wherein the acidic functionality component is in the form of a silica-alumina support.

9. The process of claim 8 wherein the silica content is less than about 50 wt%, e.g., in a range of from 15 to 30 wt%.

10. The process of any one of claims 1 to 9 wherein the feed is, or comprises, a C5+ material derived from a non-shifting Fischer-Tropsch process.

11. The process of any one of claims 1 to 9 wherein the feed is a slack wax having a boiling point in the range of 315-566 °C (600 °F - 1050 °F).

12. The process of claim 11 wherein the feed is one which has >30 ppm of sulfur.

13. The process of any of claims 1 to 9 wherein the feed is a gas field condensate.

## Patentansprüche

1. Hydroisomerisierungsverfahren, bei dem 350°F+ (176,7°C+) Kohlenwasserstoff enthaltendes Einsatzmaterial in Gegenwart von Wasserstoff und Katalysator mit saurer Funktionalität, der Kobaltkomponente, Molybdänkomponente und Hydrocrack-Unterdrückungsmittel ausgewählt aus Kupfer- und/oder Schwefelkomponente(n) umfasst, kontaktiert wird.

2. Verfahren nach Anspruch 1, bei dem das Schwefel-Hydrocrack-Unterdrückungsmittel in Form von präsulfidiertem Katalysator bereitgestellt wird oder solchen umfasst.

3. Verfahren nach Anspruch 1 oder 2, bei dem der Katalysator im Bereich von 0,01 bis 20 Gew.% Schwefel enthält.

4. Verfahren nach einem der Ansprüche 1 bis 3, bei dem auf dem Katalysator Schwefel in einer Menge im Bereich von 0,1 bis 10 Gew.% vorhanden ist.

5. Verfahren nach einem der Ansprüche 1 bis 4, bei dem dem Einsatzmaterial kontinuierlich oder periodisch Schwefelanteil(e) zugesetzt wird bzw. werden, um eine erwünschte Menge an Schwefelkomponente in dem Katalysator aufrechtzuerhalten.

6. Verfahren nach einem der Ansprüche 1 bis 5, bei dem der Kobaltgehalt des Katalysators im Bereich von 0,5 bis 20 Gew.% liegt.

7. Verfahren nach einem der Ansprüche 1 bis 6, bei dem der Molybdängehalt des Katalysators im Bereich von 1,0 bis 20 Gew.% liegt.

8. Verfahren nach einem der Ansprüche 1 bis 7, bei dem die Komponente mit saurer Funktionalität in Form eines Siliciumdioxid-Aluminiumoxid-Trägers vorliegt.

9. Verfahren nach Anspruch 8, bei dem der Siliciumdioxidgehalt weniger als etwa 50 Gew.% beträgt, z. B. im Bereich von 15 bis 30 Gew.% liegt.

10. Verfahren nach einem der Ansprüche 1 bis 9, bei dem das Einsatzmaterial C₅₊-Material ist oder umfasst, das von einem nicht Gleichgewichts-verschiebenden Fischer-Tropsch-Verfahren abgeleitet ist.

11. Verfahren nach einem der Ansprüche 1 bis 9, bei dem das Einsatzmaterial ein Rohparaffin mit einem Siedepunkt im Bereich von 315 bis 566°C (600 bis 1050°F) ist.

12. Verfahren nach Anspruch 11, bei dem das Einsatzmaterial eines ist, das > 30 ppm Schwefel aufweist.

13. Verfahren nach einem der Ansprüche 1 bis 9, bei dem das Einsatzmaterial ein Gasfeldkondensat ist.

## Revendications

1. Procédé d'hydroisomérisation comprenant la mise en contact d'une charge contenant des hydrocarbures à 176,7°C+ (350°F+) en présence d'hydrogène et d'un catalyseur ayant une fonctionnalité acide et comprenant un composant de cobalt, un composant de molybdène et un suppresseur d'hydrocraquage choisi parmi un ou des composants de cuivre et/ou de soufre.

2. Procédé selon la revendication 1, dans lequel le suppresseur d'hydrocraquage de soufre se présente sous la forme d'un catalyseur présulfuré ou comprend un tel catalyseur.

3. Procédé selon la revendication 1 ou la revendication 2, dans lequel le catalyseur contient une proportion de soufre dans une plage de 0,01 à 20% en poids.

4. Procédé selon l'une quelconque des revendications 1 à 3, dans lequel du soufre est présent sur le catalyseur en quantité dans une plage de 0,1 à 10% en poids.

5. Procédé selon l'une quelconque des revendications 1 à 4, dans lequel un radical soufre est ajouté à la charge, de manière continue ou périodique, pour maintenir une quantité désirée de composant de soufre dans le catalyseur.

6. Procédé selon l'une quelconque des revendications 1 à 5, dans lequel la teneur en cobalt du catalyseur se situe dans la plage de 0,5 à 20% en poids.

7. Procédé selon l'une quelconque des revendications 1 à 6, dans lequel la teneur en molybdène du catalyseur se situe dans la plage de 1,0 à 20% en poids.

8. Procédé selon l'une quelconque des revendications 1 à 7, dans lequel le composant de fonctionnalité acide se présente sous la forme d'un support de silice-alumine.

9. Procédé selon la revendication 8, dans lequel la teneur en silice est inférieure à environ 50% en poids, par exemple, dans la plage de 15 à 30% en poids.

10. Procédé selon l'une quelconque des revendications 1 à 9, dans lequel la charge est, ou comprend, un matériau en C₅+ dérivé d'un procédé de Fischer-Tropsch sans déplacement.

11. Procédé selon l'une quelconque des revendications 1 à 9, dans lequel la charge est une paraffine brute non déshuilée ayant un point d'ébullition dans la plage de 315 à 566 °C (600 à 1050°F).

12. Procédé selon la revendication 11, dans lequel la charge est une charge qui a plus de 30 ppm de soufre.

13. Procédé selon l'une quelconque des revendications 1 à 9, dans lequel la charge est un condensat de champ de gaz.
